# EUROPEAN PATENT APPLICATION

(11) **EP 2 390 652 A1**
(43) Date of publication of application: **30.11.2011**
(21) Application number: 10733328.8
(22) Date of filing: 15.01.2010
(51) Int. Cl.: G01N 15/14, G01N 21/64, G01N 21/78

(54) **FLUORESCENCE DETECTION DEVICE AND FLUORESCENCE DETECTION METHOD**

(30) Priority: 23.01.2009 JP 2009012460
(71) Applicant: Mitsui Engineering & Shipbuilding Co., Ltd., Chuo-ku Tokyo 104-8439 (JP)
(72) Inventor: HOSHISHIMA, Kazuteru, Tamano-shi Okayama 706-8651 (JP)
(74) Representative: Hards, Andrew
(86) International application number: PCT/JP2010/000202
(87) International publication number: WO 2010/084720

(57) **Abstract**

In order to remove autofluorescence emitted by a measurement object, fluorescence of the measurement object within a first wavelength band is first received. The first wavelength band is set so that the intensity of fluorescence emitted by the measurement object irradiated with intensity-modulated laser light is higher than that of autofluorescence emitted by the measurement object irradiated with the laser light. Then, the autofluorescence within a second wavelength band different from the first wavelength band is received. A generated fluorescent signal of the first fluorescence and a generated fluorescent signal of the autofluorescence are mixed with a modulation signal for modulating the laser light to produce first fluorescence data and autofluorescence data, respectively. The autofluorescence data is multiplied by a predetermined constant, and the thus obtained result is subtracted from the first fluorescence data to produce third fluorescence data. The third fluorescence data is used to calculate a fluorescence intensity.

## Description

### TECHNICAL FIELD

The present invention relates to a device and a method for detecting fluorescence by receiving fluorescence emitted by a measurement object irradiated with laser light and processing fluorescent signals simultaneously obtained.

### BACKGROUND ART

In the medical and biological fields, flow cytometers are widely used. A flow cytometer analyzes the type, frequency, and characteristics of a measurement object such as cells or genes by allowing a photoelectric converter such as a photomultiplier or an avalanche photodiode to receive fluorescence emitted by the measurement obj ect irradiated with laser light.

More specifically, in a flow cytometer, a suspension liquid containing a measurement object, such as a biological material (e.g., cells, DNA, RNA, enzymes, or proteins), labeled with a fluorescent reagent is allowed to flow through a tube together with a sheath liquid flowing under pressure at a speed of about 10 m/s or less so that a laminar sheath flow is formed. The measurement object in the laminar sheath flow is irradiated with laser light, and fluorescence emitted by a fluorochrome attached to the measurement object is received and identified, through which the biological material is identified using the fluorescence as a label.

Such a flow cytometer can measure the relative amounts of, for example, DNA, RNA, enzymes, proteins etc. contained in a cell, and also can quickly analyze their functions. Further, a cell sorter or the like is used to identify a specific type of cell or chromosome based on fluorescence and selectively and quickly collect only the identified specific cells or chromosomes alive.
The use of such a cell sorter is required to quickly identify more kinds of measurement objects with high accuracy based on information about fluorescence.

For example, when a sample such as a cell or an artificially-produced microbead is measured, information about fluorescence (fluorescence color or fluorescence intensity) emitted by a fluorochrome used to label the sample is of interest. However, when the sample is irradiated with laser light, fluorescence is emitted not only by the fluorochrome used as a label but also by the sample itself, such as a cell or a microbead, or a buffer solution itself in which the samples are suspended. Fluorescence emitted by the sample itself or the buffer solution has a broad spectrum, and its wavelength band often overlaps with the wavelength band of fluorescence emitted by the fluorochrome. Therefore, fluorescence emitted by the sample itself or the buffer solution is autofluorescence that should be removed.
Autofluorescence is usually much lower in fluorescence intensity than fluorescence emitted by a fluorochrome, but in some cases, has a very high intensity. In this case, when the fluorescence intensity of autofluorescence remains sufficiently lower than that of fluorescence emitted by the fluorochrome, the fluorescence emitted by the fluorochrome can be measured with a sufficiently high S/N ratio. However, when the fluorescence intensity of fluorescence emitted by the fluorochrome is not sufficiently high, the S/N ratio is reduced and therefore it is difficult to measure the fluorescence. Even when autofluorescence is low in intensity, the same problem will arise if fluorescence emitted by the fluorochrome is also low in intensity.

Patent Document 1 describes the following method for calculating fluorescence intensity.
A labeled sample is irradiated with laser light whose intensity is time-modulated at a predetermined frequency, and fluorescence emitted by the labeled sample is received by two or more detection sensors corresponding to different light-receiving wavelength bands to collect detected values containing phase information from each of the detection sensors. A correction transformation matrix is produced using parameters of a transfer function defined when it is assumed that fluorescence emitted by the labeled sample irradiated with laser light is a relaxation response of a first-order lag system. A set of the detected values containing phase information collected from each of the detection sensors is represented as a vector, and the fluorescence intensity of fluorescence emitted by the labeled sample is determined by applying an inverse matrix produced from the produced correction transformation matrix to the vector.

### PRIOR ART DOCUMENT

### Patent Document

Patent Document 1: Japanese Patent Application Laid-Open No. 2007-127415

According to the above method, it is possible to accurately calculate a fluorescence intensity, but it is necessary to produce a correction transformation matrix and calculate an inverse matrix of the matrix. Therefore a fluorescence intensity is determined quickly and easily.

### DISCLOSURE OF THE INVENTION

### Problems to be Solved by the Invention

In order to solve the above problem, it is an object of the present invention to provide a fluorescence detecting device and a fluorescence detecting method, where fluorescence intensity is determined quickly and easily by receiving fluorescence emitted by a measurement object irradiated with laser light and processing fluorescent signals simultaneously obtained.

### Means for Solving the Problems

One aspect of the present invention provides a device for detecting fluorescence by receiving fluorescence emitted by a measurement object irradiated with laser light and processing a fluorescent signal of the received fluorescence, the device including:
(A) a light source unit operable to output, as irradiation light with which the measurement object is irradiated, laser light having a wavelength for exciting the measurement obj ect to emit fluorescence, while modulating an intensity of the laser light at a predetermined frequency;
(B) a light-receiving unit that includes a first light-receiving element and a second light-receiving element, wherein
   the first light-receiving element is operable to receive first fluorescence, which is emitted by the measurement object irradiated with the irradiation light, within a first wavelength band corresponding to the first fluorescence so that an intensity of the first fluorescence is higher than that of second fluorescence emitted by the measurement obj ect irradiated with the laser light and operable to output a first fluorescent signal and,
   the second light-receiving element is operable to receive the second fluorescence, which is emitted by the measurement object, within a second wavelength band different from the first wavelength band and operable to output a second fluorescent signal;
(C) a first processing unit operable to produce, by mixing the outputted first fluorescent signal with a modulation signal for modulating an intensity of the laser light at the frequency, first fluorescence data containing a phase delay of the first fluorescent signal with respect to the modulation signal and an intensity amplitude of the first fluorescent signal and also operable to produce, by mixing the outputted second fluorescent signal with the modulation signal, second fluorescence data containing a phase delay of the second fluorescent signal with respect to the modulation signal and an intensity amplitude of the second fluorescent signal; and
(D) a second processing unit that includes a fluorescence removing unit and a fluorescence intensity calculating unit, wherein
   the fluorescence removing unit is operable to produce third fluorescence data by subtracting, from the first fluorescence data, a result obtained by multiplying the produced second fluorescence data by a predetermined constant and,
   the fluorescence intensity calculating unit is operable to calculate a fluorescence intensity of the first fluorescence using the produced third fluorescence data.

Another aspect of the present invention provides a method for detecting fluorescence by receiving fluorescence emitted by a measurement object irradiated with laser light and processing a fluorescent signal of the received fluorescence, the method including the steps of:
(E) outputting, as irradiation light with which the measurement object is irradiated, laser light having a wavelength for exciting the measurement object to emit fluorescence, while modulating an intensity of the laser light at a predetermined frequency;
(F) receiving first fluorescence, which is emitted by the measurement object irradiated with the irradiation light, within a first wavelength band corresponding to the first fluorescence so that an intensity of the first fluorescence is higher than that of second fluorescence emitted by the measurement object irradiated with the laser light to generate a first fluorescent signal, and receiving the second fluorescence, which is emitted by the measurement object, within a second wavelength band different from the first wavelength band to generate a second fluorescent signal;
(G) producing, by mixing the generated first fluorescent signal with a modulation signal for modulating an intensity of the laser light at the frequency, first fluorescence data containing a phase delay of the first fluorescent signal with respect to the modulation signal and an intensity amplitude of the first fluorescent signal, and producing, by mixing the generated second fluorescent signal with the modulation signal, second fluorescence data containing a phase delay of the second fluorescent signal with respect to the modulation signal and an intensity amplitude of the second fluorescent signal; and
(H) calculating a fluorescence intensity of the first fluorescence using third fluorescence data which is produced by subtracting, from the first fluorescence data, a result obtained by multiplying the produced second fluorescence data by a predetermined constant.

### EFFECTS OF THE INVENTION

The fluorescence detecting device and the fluorescence detecting method according to the above aspects of the present invention are capable of calculating the fluorescence intensity of the first fluorescence more quickly and easily than ever before.

### BRIEF DESCRIPTION OF THE DRAWINGS

FIG. 1 is a schematic diagram illustrating the structure of a flow cytometer that employs a fluorescence detecting device according to the present invention using intensity-modulated laser light.
FIG. 2 is a schematic diagram illustrating the structure of one example of a light source unit used in the flow cytometer illustrated in FIG. 1.
FIG. 3 is a schematic diagram illustrating the structure of one example of a light-receiving unit used in the flow cytometer illustrated in FIG. 1.
FIG. 4 is a diagram illustrating the relationship among the spectrum S of fluorescence emitted by a fluorescent protein, the wavelength of laser light L, a first wavelength band, and a second wavelength band.
FIG. 5 is a schematic diagram illustrating the structure of a main part of the flow cytometer illustrated in FIG. 1, which mainly illustrates a control/processing unit of the flow cytometer.
FIG. 6 is a schematic diagram illustrating the structure of one example of an analyzing device used in the flow cytometer illustrated in FIG. 1.
FIG. 7 is a schematic diagram for explaining a method for removing autofluorescence.

### DESCRIPTION OF THE REFERENCE NUMERALS

- 10: flow cytometer
- 12: sample
- 20: signal processing device
- 22: laser light source unit
- 22a: light source
- 23a, 26b: dichroic mirror
- 23b, 26a: lens system
- 24, 26: light-receiving unit
- 26c₁, 26c₂: band-pass filter
- 27a, 27b: photoelectric converter
- 28: control/processing unit
- 30: tube
- 32: collection vessel
- 34: laser driver
- 48a, 48b: power splitter
- 40: signal generation unit
- 42: signal processing unit
- 44: system controller
- 46: oscillator
- 49, 62: low-pass filter
- 50, 52, 54a, 54b, 64: amplifier
- 58a, 58b: IQ mixer
- 62: low-pass filter
- 66: A/D converter
- 80: analyzing device
- 81: CPU
- 82: memory
- 83: analyzing unit
- 86: autofluorescence removing unit
- 90: fluorescence intensity calculating unit
- 92: phase delay calculating unit
- 94: fluorescence relaxation time calculating unit

### DESCRIPTION OF EMBODIMENTS

Hereinbelow, the present invention will be described in detail based on a flow cytometer preferably employing a fluorescence detecting device according to the present invention for detecting fluorescence emitted by irradiation with intensity-modulated laser light.

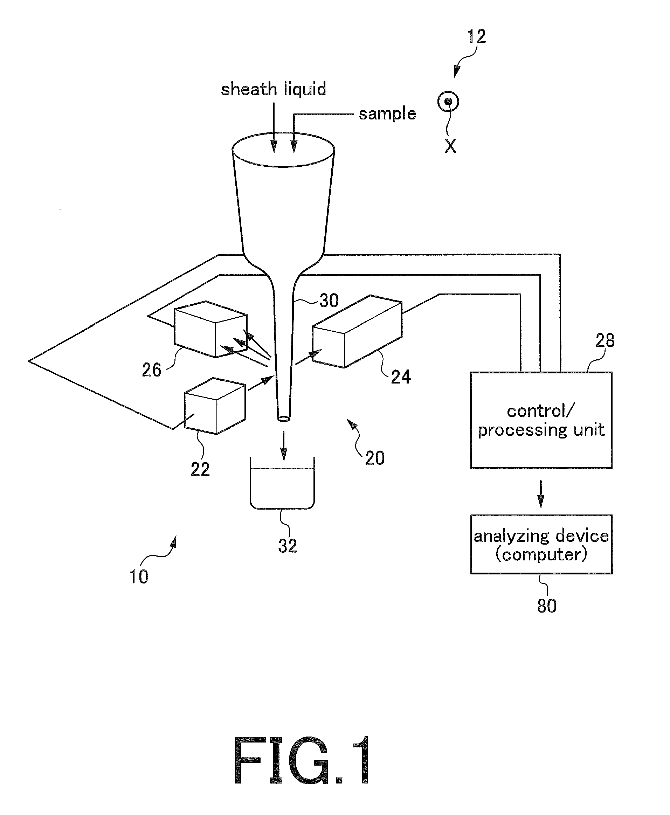

FIG. 1 is a schematic diagram illustrating the structure of a flow cytometer 10 employing a fluorescence detecting device according to the present invention for detecting fluorescence emitted by irradiation with intensity-modulated laser light.

The flow cytometer 10 includes a signal processing device 20 and an analyzing device (computer) 80. The signal processing device 20 detects and processes a fluorescent signal of fluorescence emitted by a sample 12, which is a measurement object, by irradiation with laser light. The analyzing device (computer) 80 calculates a fluorescence intensity and a fluorescence relaxation time from processing results obtained by the signal processing device 20.

The following description is made with reference to a case where a cell having a fluorescent protein X attached thereto is used as the sample 12. A buffer solution containing the samples 12 is allowed to flow through a flow cell together with a sheath liquid to form a laminar sheath flow. The sample 12 in the laminar sheath flow is irradiated with laser light. However, in the present invention, the sample 12 may be replaced with a cell having two or more fluorochromes attached thereto. Also in this case, autofluorescence emitted by, for example, the cell or the buffer solution can be removed from fluorescence emitted by the two or more fluorochromes.

The signal processing device 20 includes a laser light source unit 22, light-receiving units 24 and 26, a control/processing unit 28, and a tube 30.
The control/processing unit 28 includes a control unit that modulates the intensity of laser light emitted from the laser light source unit 22 at a predetermined frequency and a signal processing unit that processes a fluorescent signal from the sample 12. The tube 30 allows an amount of samples 12 to flow individually therethrough together with a sheath liquid forming a high-speed flow so that a laminar sheath flow is formed. A collection vessel 32 is provided at the outlet of the tube 30. The flow cytometer 10 may include a cell sorter for quickly separating a biological material, such as specific cells, among the samples 12 after irradiation with laser light to collect the biological material in different collection vessels.

The laser light source unit 22 is a unit that emits laser light having a predetermined wavelength, e.g., laser light of λ = 408 nm. A lens system is provided so that the laser light is focused on a predetermined position in the tube 30, and the focus position is defined as a measurement point at which the sample 12 is measured.

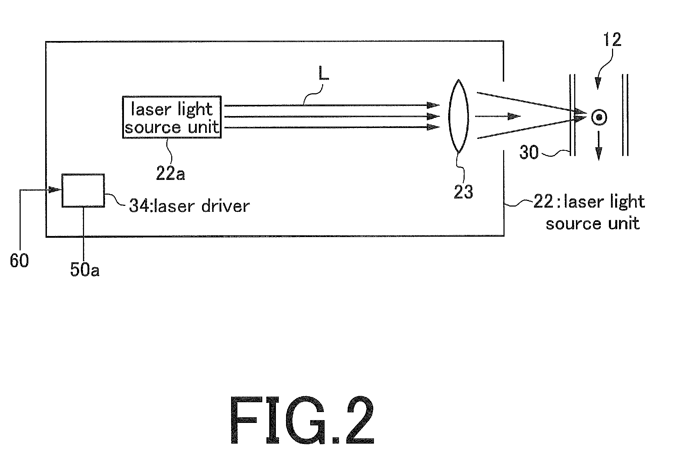

FIG. 2 is a diagram illustrating one example of the structure of the laser light source unit 22.
The laser light source unit 22 is a unit that emits intensity-modulated laser light having a wavelength within a visible light band.
The laser light source unit 22 includes a light source (laser diode) 22a. The light source 22a emits laser light having a wavelength of 408 nm as CW (continuous-wave) laser light L while modulating the intensity of the CW laser light L at a predetermined frequency. The laser light source unit 22 further includes a lens system 23 and a laser driver 34.
The lens system 23 focuses the laser light L on the measurement point in the tube 30. The laser driver 34 drives the laser light source unit 22.

As the light source that emits the laser light L, for example, a semiconductor laser is used. The laser light L has an output of, for example, about 5 to 100 mW. A frequency (modulation frequency) used to modulate the intensity of the laser light L has a periodic time slightly longer than a fluorescence relaxation time, and is, for example, 10 to 200 MHz.

The laser light source unit 22 oscillates at a predetermined wavelength band so that a fluorochrome is excited by the laser light L and emits fluorescence of a specific wavelength band. The fluorescent protein X to be excited by the laser light L is attached (bound) to the cell. When passing through the measurement point in the tube 30, the sample 12 is irradiated with the laser light L at the measurement point, and then the fluorescent protein X emits fluorescence at a specific wavelength.

The light-receiving unit 24 is arranged so as to be opposed to the laser light source unit 22 with the tube 30 being provided therebetween. The light-receiving unit 24 is equipped with a photoelectric converter that detects forward scattering of laser light caused by the sample 12 passing through the measurement point and outputs a detection signal indicating the passage of the sample 12 through the measurement point. The detection signal outputted from the light-receiving unit 24 is supplied to the control/processing unit 28 and the analyzing device 80 and is used as a trigger signal for announcement of the timing of passage of the sample 12 through the measurement point in the tube 30 and as an ON signal for controlling the start of processing or an OFF signal.

On the other hand, the light-receiving unit 26 is arranged in a direction perpendicular to a direction in which laser light emitted from the laser light source unit 22 travels and to a direction in which the samples 12 move in the tube 30. The light-receiving unit 26 is equipped with two or more photoelectric converters that receive fluorescence emitted by the sample 12 irradiated with laser light at the measurement point.

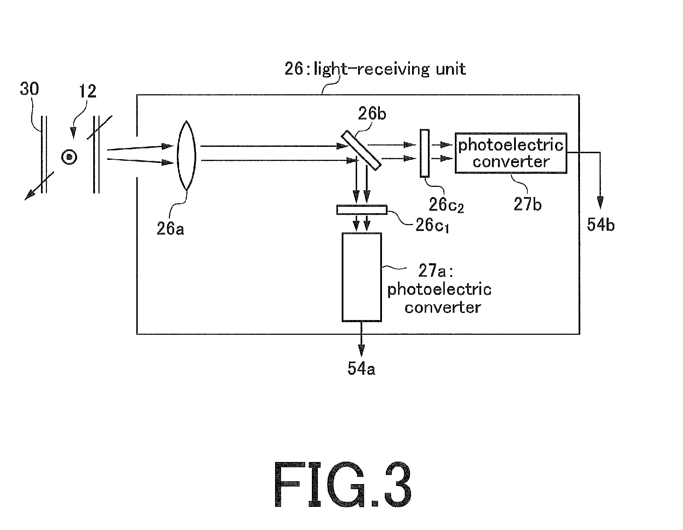

FIG. 3 is a schematic diagram illustrating the structure of one example of the light-receiving unit 26.

The light-receiving unit 26 illustrated in FIG. 3 includes a lens system 26a that focuses fluorescent signals from the sample 12, a dichroic mirror 26b, band-pass filters 26c₁ and 26c₂, and photoelectric converters (light-receiving elements) 27a and 27b such as photomultipliers.
The lens system 26a is configured to focus fluorescence received by the light-receiving unit 26 on the light-receiving surfaces of the photoelectric converters 27a and 27b.
The dichroic mirror 26b is a mirror that reflects fluorescence of wavelengths within a predetermined wavelength band but transmits fluorescence of wavelengths outside the predetermined wavelength band. The reflection wavelength band of the dichroic mirror 26b and the transmission wavelength bands of the band-pass filters 26c₁ and 26c₂ are set so that fluorescence of a predetermined wavelength band can be received by the photoelectric converter 27a after filtering by the band-pass filter 26c₁ and fluorescence of a predetermined wavelength band can be received by the photoelectric converter 27b after filtering by the band-pass filter 26c₂.

The band-pass filter 26c₁ is provided in front of the light-receiving surface of the photoelectric converter 27a and transmits only fluorescence of a predetermined wavelength band, while the band-pass filter 26c₂ is provided in front of the light-receiving surface of the photoelectric converter 27b and transmits only fluorescence of a predetermined wavelength band. The wavelength band of fluorescence that can pass through one of the band-pass filters 26c₁ and 26c₂ is set so as to correspond to fluorescence emitted by the fluorescent protein X. For example, the predetermined transmission wavelength bands are set to a first wavelength band FL₁ ranging from 494 to 535 nm to mainly receive fluorescence emitted by the fluorescent protein X by irradiation with the laser light L of 408 nm emitted from the laser light source unit 22, and set to a second wavelength band FL_{b} ranging from 415 to 440 nm. The first wavelength band FL₁ is set so as to correspond to the fluorescent protein X so that, when the sample 12 is irradiated with the laser light L, the fluorescence intensity of fluorescence emitted by the fluorescent protein X is higher than that of autofluorescence emitted by the cell itself or the buffer solution itself. Similarly, the second wavelength band FL_{b} is set so that, when the sample 12 is irradiated with the laser light L, the fluorescence intensity of autofluorescence is higher than that of fluorescence emitted by the fluorescent protein X. It is to be noted that, as will be described later, the second wavelength band FL_{b} is preferably set to be outside the wavelength range of fluorescence emitted by the fluorescent protein X in order to effectively remove fluorescence data of autofluorescence.

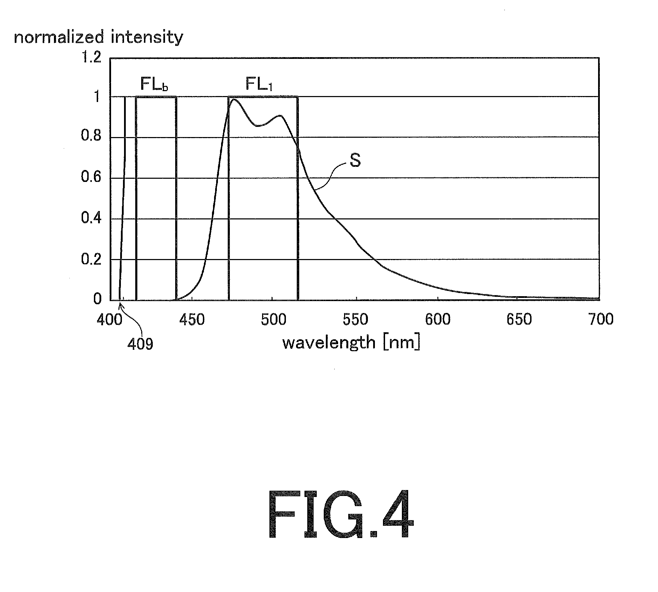

FIG. 4 illustrates one example of the relationship among the spectrum S of fluorescence emitted by the fluorescent protein X, the wavelength of the laser light L (408 nm), the first wavelength band FL₁, and the second wavelength band FL_{b}.

Autofluorescence has a very broad spectral distribution, and therefore its wavelength band overlaps with both the first wavelength band FL₁ and the second wavelength band FL_{b}. Therefore, fluorescence emitted by the fluorescent protein X and autofluorescence are received by the photoelectric converter within the first wavelength band FL₁. On the other hand, fluorescence emitted by the fluorescent protein X has a narrower spectral distribution than autofluorescence. Therefore, a wavelength range exists where the fluorescence intensity of fluorescence emitted by the fluorescent protein X dominant in the wavelength band FL₁ is lower than that of autofluorescence emitted by the cell or the buffer solution or a wavelength range exists where the wavelength range does not overlap with the wavelength band of fluorescence emitted by the fluorescent protein X. Such a wavelength range is set as the wavelength band FL_{b}.

The photoelectric converters 27a and 27b are each a light-receiving element equipped with, for example, a sensor such as a photomultiplier to convert light received by its photoelectric surface into an electric signal. Here, the emission of fluorescence to be received by each of the photoelectric converters is induced by excitation with laser light whose intensity is modulated at a predetermined frequency, and therefore a fluorescent signal outputted from each of the photoelectric converters is a signal whose intensity varies at a predetermined frequency. Such a fluorescent signal is supplied to the control/processing unit 28.

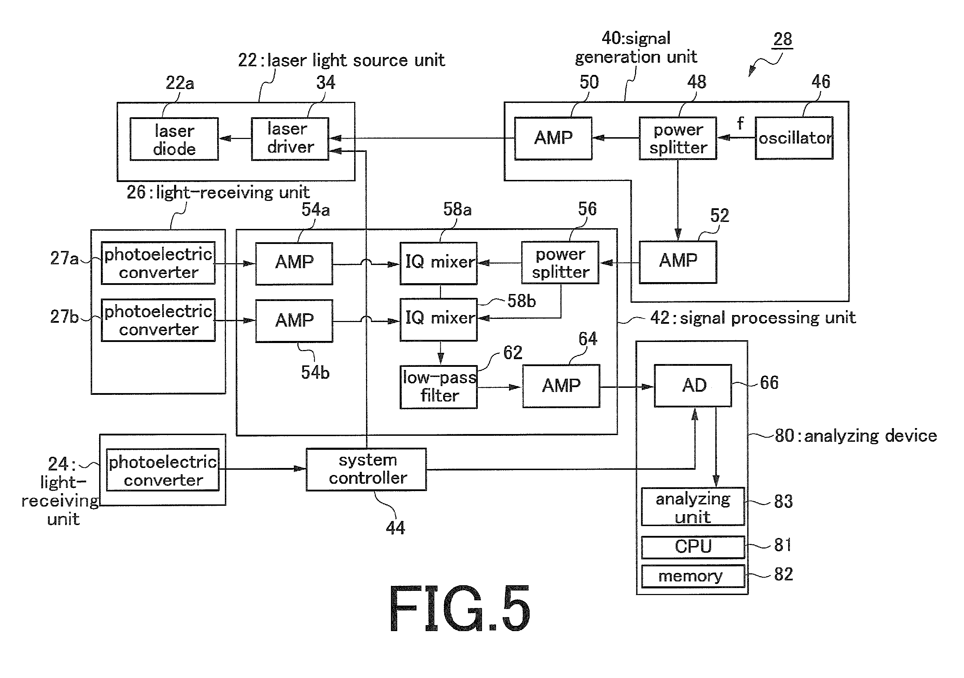

As illustrated in FIG. 5, the control/processing unit 28 includes a signal generation unit 40, a signal processing unit 42, and a system controller 44 as a control unit.
The signal generation unit 40 generates a modulation signal for modulating the intensity of the laser light L at a predetermined frequency of f.
More specifically, the signal generation unit 40 includes an oscillator 46, a power splitter 48, and amplifiers 50 and 52. The signal generation unit 40 supplies a modulation signal generated by the oscillator 46, split by the power splitter 48, and amplified by the amplifier 50 to the laser driver 34 of the laser light source unit 22, and also supplies a modulation signal split by the power splitter 48 and amplified by the amplifier 52 to the signal processing unit 42. As will be described later, the modulation signal supplied to the signal processing unit 42 is used as a reference signal for detecting fluorescent signals outputted from the photoelectric converters 27a and 27b. It is to be noted that the modulation signal is a signal with a predetermined frequency, and the frequency is set to a value in the range of 10 to 200 MHz. The oscillator 46 generates a signal with a frequency f as a modulation signal.

The signal processing unit 42 extracts, by using fluorescent signals outputted from the photoelectric converters 27a and 27b, fluorescence data of fluorescence emitted by the fluorescent protein X irradiated with laser light and fluorescence data of autofluorescence emitted by, for example, the cell. The signal processing unit 42 includes amplifiers 54a, 54b, and 64, a power splitter 56, IQ mixers 58a and 58b, and a low-pass filter 62.

The amplifier 54a amplifies a fluorescent signal outputted from the photoelectric converter 27a and the amplifier 54b amplifies a fluorescent signal outputted from the photoelectric converter 27b. Each of the IQ mixers 58a and 58b mixes the amplified fluorescent signal with the modulation signal (reference signal) that is a sinusoidal signal supplied from the signal generation unit 40.
The power splitter 56 divides the modulation signal supplied from the signal generation unit 40 into two signals so that one of the signals is sent to the IQ mixer 58a and the other signal is sent to the IQ mixer 58b.

The IQ mixer 58a is a device that mixes the fluorescent signal supplied from the photoelectric converter 27a with the modulation signal supplied from the signal generation unit 40 as a reference signal, and the IQ mixer 58b is a device that mixes the fluorescent signal supplied from the photoelectric converter 27b with the modulation signal supplied from the signal generation unit 40 as a reference signal. More specifically, each of the IQ mixers 58a and 58b multiplies the reference signal by the fluorescent signal (RF signal) to generate a signal containing a component of the fluorescent signal in phase with the modulation signal and a signal containing a component of the fluorescent signal 90 degrees phase-shifted with respect to the modulation signal. The signal containing an in-phase component is generated by mixing the modulation signal with the fluorescent signal, and the signal containing a component 90 degrees phase-shifted is generated by mixing a signal obtained by shifting the phase of the modulation signal by 90° with the fluorescent signal.

The low-pass filter 62 is a unit that filters signals generated by the IQ mixers 58a and 58b to extract low-frequency components. By performing the filtering, a component (Re component) of the fluorescent signal in phase with the modulation signal and a component (Im component) of the fluorescent signal 90 degrees phase-shifted with respect to the modulation signal are extracted as fluorescence data. The extracted Re component and Im component are amplified by the amplifier 64 and sent to the analyzing device 80. The Re component and the Im component can be obtained from both the first wavelength band and the second wavelength band corresponding to the photoelectric converter 27a and the photoelectric converter 27b. Therefore, a pair of the Re component and the Im component obtained from the first wavelength band and a pair of the Re component and the Im component obtained from the second wavelength band are sent to the analyzing device 80.

The system controller 44 controls the signal generation unit 40 to generate a modulation signal with a predetermined frequency, and further gives instructions for controlling the operations of the individual units and manages all the operations of the flow cytometer 10.

The analyzing device 80 performs A/D conversion of the Re component and the Im component supplied from the signal processing unit 42, determines, from the A/D converted Re component and the A/D converted Im component, a fluorescence intensity and a phase delay angle of fluorescence with respect to the laser light, and determines, from the phase delay angle, a fluorescence relaxation time constant (fluorescence relaxation time). More specifically, the analyzing device 80 includes an A/D converter 66 and an analyzing unit 83. The analyzing device 80 is constituted of a computer including a CPU 81 and a memory 82, and the analyzing unit 83 is configured as a software module operated by reading and executing a program stored in the memory 82. Each of units constituting the analyzing unit 83 can be, of course, provided by a dedicated circuit.

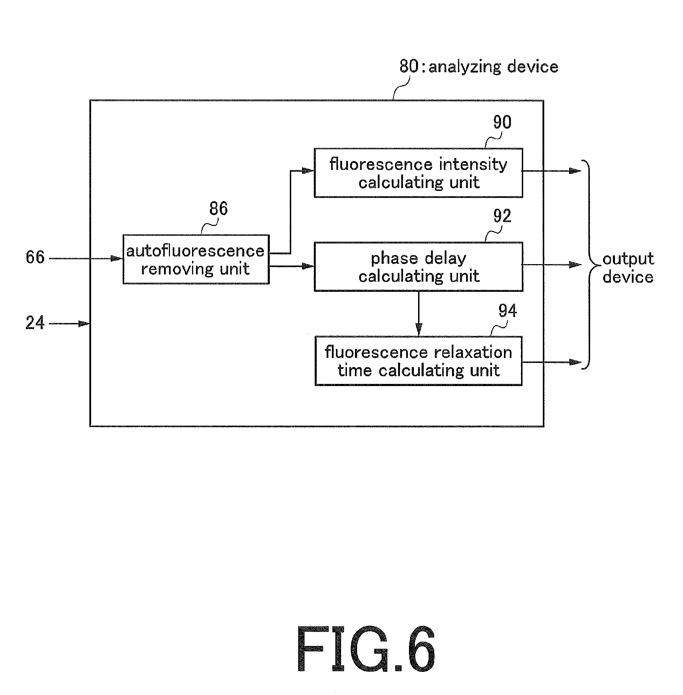

FIG. 6 is a schematic diagram illustrating the structure of the analyzing unit 83.
The analyzing unit 83 includes an autofluorescence removing unit 86, a fluorescence intensity calculating unit 90, a phase delay calculating unit 92, and a fluorescence relaxation time calculating unit 94.

The autofluorescence removing unit 86 is a unit that removes information about autofluorescence emitted by the cell itself of the sample 12 or the buffer solution itself from information about fluorescence within the first wavelength band FL₁ and the second wavelength band FL_{b} by using fluorescence data represented by a complex number having the Re component and the Im component supplied from the control unit 44. More specifically, fluorescence data within the second wavelength band FL_{b} is multiplied by a predetermined first constant (complex number), and a result obtained by the multiplication is subtracted from fluorescence data within the first wavelength band FL₁ to remove fluorescence data of autofluorescence from the fluorescence data within the first wavelength band FL₁.

The first constant is obtained by measuring the cell not having the fluorescent protein X attached thereto using the light source unit 22, the light-receiving unit 24, and the control/processing unit 28. The first constant is a ratio obtained by dividing fluorescence data of autofluorescence within the first wavelength band FL₁ emitted by the cell by fluorescence data of autofluorescence within the second wavelength band FL_{b} emitted by the cell.

For example, when the fluorescence data of autofluorescence within the first wavelength band FL₁ is represented by a complex number, a₁e^{iθ1} and the fluorescence data of autofluorescence FL_{b} within the second wavelength band is represented by a complex number, a_{b}e^{iθb}, the first constant is represented as a₁/a_{b} · e^{i(θ1 - θb)}.
Further, when fluorescence data obtained from the second wavelength band FL_{b} by measuring the sample 12 is represented by a complex number, A_{b}e^{iθb} and fluorescence data within the first wavelength band FL₁ by measuring the sample 12 is represented by a complex number A₁e^{iθ1}, A₁e^{iθ1} - a₁/a_{b} · e^{i(θ1 - θb)} · A_{b}e^{iθb} is calculated. The calculation result is fluorescence data obtained by removing fluorescence data of autofluorescence from the fluorescence data within the first wavelength band FL₁.
In this way, fluorescence data of autofluorescence within the first wavelength band FL₁ can be estimated by multiplying fluorescence data within the second wavelength band FL_{b} by measuring the sample 12 by the first constant, that is, by determining a₁/a_{b} · e^{i(θ1 - θb)} · A_{b}e^{iθb}. The first constant is stored in the memory 84 of the analyzing device 80.

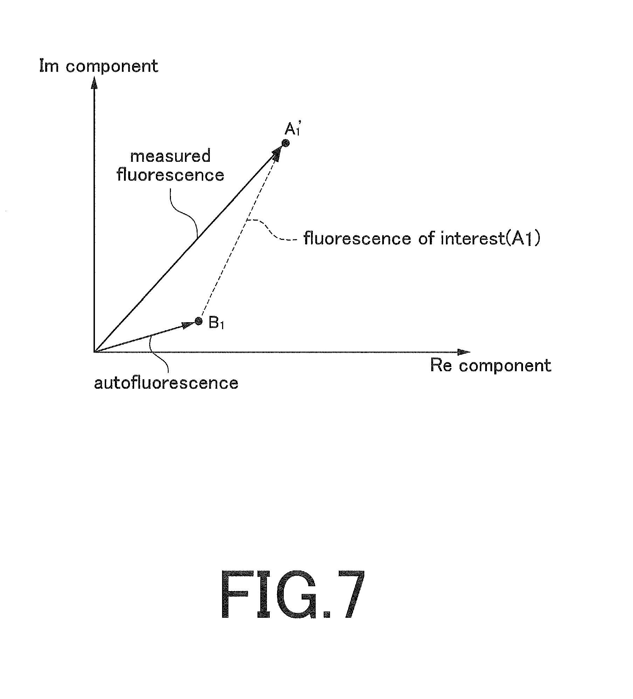

FIG. 7 is a schematic diagram for explaining a method for removing autofluorescence. In FIG. 7, the vertical axis represents the Im component, the horizontal axis represents the Re component, and fluorescence is represented by vector. Fluorescence data B₁ of autofluorescence within the first wavelength band FL₁ is obtained by multiplying fluorescence data obtained from the second wavelength band FL_{b} by the first constant. Fluorescence data A₁ of interest represented by the dotted line in FIG. 7 is calculated by subtracting the fluorescence data B₁ from fluorescence data A₁' which is measured within the first wavelength band FL₁.
The thus obtained fluorescence data, from which the fluorescence data of autofluorescence has been removed, is supplied to the fluorescence intensity calculating unit 90 and the phase delay calculating unit 92.

The fluorescence intensity calculating unit 90 is a unit that calculates a fluorescence intensity by determining the absolute value of a complex number representing the corrected fluorescence data A₁.
The phase delay calculating unit 92 is a unit that calculates the argument of a complex number representing the corrected fluorescence data A₁ (tan⁻¹(Im component of fluorescence data/Re component of fluorescence data)) as a phase delay θ.
The fluorescence relaxation time calculating unit 94 is a unit that calculates a fluorescence relaxation time τ using the phase delay θ calculated by the phase delay calculating unit 92 according to the formula: τ = 1/(2πf) · tan(θ), where f is a frequency used to modulate the intensity of the laser light L. The reason why the fluorescence relaxation time τ can be calculated according to the formula: τ = 1/(2πf) · tan(θ) is that a fluorescence phenomenon shifts according to a first-order relaxation process.
The thus calculated fluorescence intensity, phase delay θ, and fluorescence relaxation time τ are outputted as result information to a printer or display (not illustrated). The result information is a result measured every time each of samples 12 passes through the measurement point in the tube 30, and the measurement result is used for statistical processing.
The flow cytometer 10 has such a structure as described above.

Hereinbelow, a method for detecting fluorescence using the flow cytometer 10 will be described.
First, the laser light L having a wavelength absorbed by the fluorescent protein X is prepared so that fluorescence whose intensity is higher than that of autofluorescence is emitted from the fluorescent protein X. The light source unit 22 emits the laser light L while modulating the intensity of the laser light L at a frequency off.

Then, the light-receiving unit 26 receives fluorescence within the first wavelength band FL₁ corresponding to the fluorescent protein X so that the fluorescence intensity of fluorescence emitted by the fluorescent protein X irradiated with irradiation light is higher than that of autofluorescence, and also receives autofluorescence within the second wavelength band FL_{b}, which is different from the first wavelength band FL₁. The autofluorescence is emitted by, for example, the cell. Then, the light-receiving unit 26 outputs a fluorescent signal corresponding to the first wavelength band FL₁ and a fluorescent signal corresponding to the second wavelength band FL_{b}.
In the control/processing unit 28, each of the outputted fluorescent signal is mixed with the modulation signal with a frequency f to produce fluorescence data A1' containing the phase delay of the fluorescent signal with respect to the modulation signal and the intensity amplitude of the fluorescent signal, and the fluorescent signal corresponding to the second wavelength band FL_{b} is mixed with the modulation signal with a frequency f to produce fluorescence data B₁' containing the phase delay of the fluorescent signal with respect to the modulation signal and the intensity amplitude of the fluorescent signal.

The fluorescence data A₁' and the fluorescence data B₁' produced by the control/processing unit 28 are sent to the analyzing device 80 to perform processing for removing autofluorescence. In this processing, the fluorescence data B₁' is multiplied by the first constant (complex number) to obtain fluorescence data B₁, and the fluorescence data B₁ is subtracted from the fluorescence data A₁' to obtain fluorescence data A₁ from which autofluorescence has been removed. The fluorescence data A₁ is sent to the fluorescence intensity calculating unit 90 and the phase delay calculating unit 92 to calculate a fluorescence intensity and a phase delay θ. Further, the fluorescence relaxation time calculating unit 94 calculates a fluorescence relaxation time τ using the phase delay θ calculated by the phase delay calculating unit 92.

It is to be noted that the first constant is a ratio (complex number) obtained by dividing fluorescence data of autofluorescence within the first wavelength band FL₁ emitted by the cell by fluorescence data of autofluorescence within the second wavelength band FL_{b} emitted by the cell. The fluorescence data are previously determined by measuring the cell not having the fluorescent protein X attached (bound) thereto with the flow cytometer 10.

As has been described above, in the fluorescence detecting device and the fluorescence detecting method according to the present invention, a first fluorescent signal is generated by receiving first fluorescence and second fluorescence within a first wavelength band FL₁ set so that the intensity of the first fluorescence is higher than that of the second fluorescence, and a second fluorescent signal is generated by receiving the second fluorescence within a second fluorescence band FL_{b} different from the first wavelength band FL₁. Fluorescence data of autofluorescence within the first wavelength band FL₁ is estimated by multiplying fluorescence data within the second wavelength band FL_{b} by measuring the sample 12 by a first constant. Therefore, autofluorescence can be quickly and easily removed simply by subtracting the fluorescence data of autofluorescence.
Particularly, when the second fluorescence is autofluorescence emitted by a particle to be measured and having a broad spectral distribution over a wide wavelength range, autofluorescence can be quickly and easily removed from the first fluorescence.

Although the fluorescence detecting device and the fluorescence detecting method according to the present invention have been described above in detail, the present invention is not limited to the above embodiment, and it should be understood that various changes and modifications may be made without departing from the scope of the present invention.

## Claims

1. A device for detecting fluorescence by receiving fluorescence emitted by a measurement object irradiated with laser light and processing a fluorescent signal of the received fluorescence, the device comprising:
a light source unit operable to output, as irradiation light with which the measurement object is irradiated, laser light having a wavelength for exciting the measurement object to emit fluorescence, while modulating an intensity of the laser light at a predetermined frequency;
a light-receiving unit provided with a first light-receiving element and a second light-receiving element, wherein
the first light-receiving element is operable to receive first fluorescence within a first wavelength band corresponding to the first fluorescence such that an intensity of the first fluorescence is higher than that of second fluorescence emitted by the measurement object irradiated with the laser light, and to output a first fluorescent signal, the first fluorescence being emitted by the measurement object irradiated with the irradiation light and,
the second light-receiving element is operable to receive the second fluorescence within a second wavelength band different from the first wavelength band and operable to output a second fluorescent signal, the second fluorescence being emitted by the measurement object;
a first processing unit operable to produce, by mixing the outputted first fluorescent signal with a modulation signal for modulating an intensity of the laser light at the frequency, first fluorescence data containing a phase delay of the first fluorescent signal with respect to the modulation signal and an intensity amplitude of the first fluorescent signal, and also operable to produce, by mixing the outputted second fluorescent signal with the modulation signal, second fluorescence data containing a phase delay of the second fluorescent signal with respect to the modulation signal and an intensity amplitude of the second fluorescent signal; and
a second processing unit that includes a fluorescence removing unit and a fluorescence intensity calculating unit, wherein
the fluorescence removing unit is operable to produce third fluorescence data by subtracting, from the first fluorescence data, a result obtained by multiplying the produced second fluorescence data by a predetermined constant and,
the fluorescence intensity calculating unit is operable to calculate a fluorescence intensity of the first fluorescence using the produced third fluorescence data.

2. The device according to claim 1, wherein the measurement object is composed of a measurement particle and a fluorochrome attached to the measurement particle, and
wherein the first fluorescence is fluorescence emitted by the fluorochrome and the second fluorescence is autofluorescence emitted by the measurement particle or autofluorescence emitted by a solution in which the measurement particle is suspended.

3. The device according to claim 2, wherein the second wavelength band is set to be outside a wavelength range of the first fluorescence.

4. The device according to claim 2 or 3, wherein the constant used in the fluorescence removing unit is a ratio obtained by dividing fluorescence data of autofluorescence within the first wavelength band emitted by the measurement particle by fluorescence data of autofluorescence within the second wavelength band emitted by the measurement particle, the fluorescence data being obtained by measuring the measurement particle having no fluorochrome attached thereto using the light source unit, the light-receiving unit, and the first processing unit.

5. The device according to any one of claims 1 to 4, wherein the second processing unit, in addition to calculating a fluorescence intensity, includes a fluorescence relaxation time calculating unit operable to calculate a fluorescence relaxation time of the first fluorescence using the third fluorescence data.

6. A method for detecting fluorescence by receiving fluorescence emitted by a measurement object irradiated with laser light and processing a fluorescent signal of the received fluorescence, the method comprising the steps of:
outputting, as irradiation light with which the measurement obj ect is irradiated, laser light having a wavelength for exciting the measurement object to emit fluorescence, while modulating an intensity of the laser light at a predetermined frequency;
receiving first fluorescence within a first wavelength band corresponding to the first fluorescence such that an intensity of the first fluorescence is higher than that of second fluorescence emitted by the measurement object irradiated with the laser light and generating a first fluorescent signal, and receiving the second fluorescence within a second wavelength band different from the first wavelength band and generating a second fluorescent signal, wherein the first fluorescence is emitted by the measurement obj ect irradiated with the irradiation light and the second fluorescence is emitted by the measurement object;
producing, by mixing the generated first fluorescent signal with a modulation signal for modulating an intensity of the laser light at the frequency, first fluorescence data containing a phase delay of the first fluorescent signal with respect to the modulation signal and an intensity amplitude of the first fluorescent signal, and producing, by mixing the generated second fluorescent signal with the modulation signal, second fluorescence data containing a phase delay of the second fluorescent signal with respect to the modulation signal and an intensity amplitude of the second fluorescent signal; and
calculating a fluorescence intensity of the first fluorescence using third fluorescence data which is produced by subtracting, from the first fluorescence data, a result obtained by multiplying the produced second fluorescence data by a predetermined constant.

7. The method according to claim 6, wherein the measurement object is composed of a measurement particle and a fluorochrome attached to the measurement particle, and
wherein the first fluorescence is fluorescence emitted by the fluorochrome and the second fluorescence is autofluorescence emitted by the measurement particle or autofluorescence emitted by a solution in which the measurement particle is suspended.

8. The method according to claim 7, wherein the second wavelength band is set to be outside a wavelength range of the first fluorescence.

9. The method according to claim 7 or 8, wherein the constant is determined by a processing method using the measurement particle having no fluorochrome attached thereto, and
wherein the processing method includes the steps of:
outputting, as irradiation light with which the measurement obj ect is irradiated, the laser light while modulating an intensity of the laser light at the frequency;
receiving the autofluorescence within the first wavelength band to generate a first autofluorescent signal and receiving the autofluorescence within the second wavelength band to generate a second autofluorescent signal;and
calculating, as the constant, a ratio of the generated first autofluorescent signal to the generated second autofluorescent signal.

10. The method according to any one of claims 6 to 9, further comprising, in addition to calculating a fluorescence intensity, calculating a fluorescence relaxation time of the first fluorescence using the third fluorescence data.
